# EUROPEAN PATENT APPLICATION

(11) **EP 4 324 810 A1**
(43) Date of publication of application: **21.02.2024**
(21) Application number: 23185978.6
(22) Date of filing: 18.07.2023
(51) Int. Cl.: C07C 2/72, C07C 15/02, C07C 7/04, C07C 7/00, C07C 7/10

(54) **PROCESS FOR THE SYNTHESIS OF MIBT**

(30) Priority: 02.08.2022 IN 202221044199
(71) Applicant: Vinati Organics Limited, Mumbai 400051 (IN)
(72) Inventor: SARAF, Vinod Kumar, 400051 Mumbai (IN)
(74) Representative: Meissner Bolte Partnerschaft mbB

(57) **Abstract**

The present disclosure discloses a process (100) for the synthesis of MIBT capable of producing an alkyl-substituted aromatic hydrocarbon in a highly selective and high yield with an excellent conversion rate of an aromatic hydrocarbon as a reaction substrate. The process further employs a catalyst slurry that comprises a catalyst carrier, an alkali metal catalyst, and an organic solvent. The side-chain alkylation of m-xylene was carried out using propylene in the presence of a catalyst slurry.

## Description

### CROSS-REFERENCE TO RELATED APPLICATIONS AND PRIORITY

The present application claims priority from the Indian patent application no. 202221044199 filed on 02 August 2022.

### TECHNICAL FIELD

The present invention disclosure relates to a process for the alkylation of aromatic compounds. More specifically, this disclosure relates to a process for the synthesis of m-isobutyl toluene (MIBT).

### BACKGROUND

Alkyl-substituted aromatic hydrocarbons such as m-isobutyl toluene (MIBT) are useful as intermediate raw materials for organic compounds such as pharmaceuticals and fragrances, and methods for producing alkyl-substituted aromatic hydrocarbons by introducing alkyl groups into aromatic hydrocarbons have been studied. In the alkylation process, a succession of reactions may occur whereby the alkylated products comprise a mixture of mono, di- and tri-alkylated compounds, up to the limit of substitutable hydrogen atoms on the nucleus. The introduction of the original alkyl group activates the nucleus for further substitution so that the mono-alkyl benzenes are subject to further alkylation to an extent dependent on the catalyst, the olefinic alkylating agent, and the reaction conditions.

The known methods for producing alkyl substituted aromatic hydrocarbons such as MIBT selectively involve the side chain alkylation of m-xylene with propylene over a solid catalyst. In the existing knowledge of the art, known processes also suffer from the formation of undesirable hydrocarbon by-products, and catalyst residue and therefore substantially increase the reaction time of the m-xylene alkylation and further reduce the yield.

It has been found that deleterious effects on downstream processes of the presence of free-radical reaction inhibitors in the m-xylene stream may be reduced by treatment of the catalyst with an inert atmosphere and a reducing atmosphere, thereby reducing the presence of undesirable catalyst impurities that are believed to produce free-radical reaction inhibitors.

However, there are various drawbacks in the available methods. The available methods do not use the specific catalyst to enhance the production of MIBT, carrier drying to improve the coating, ratio optimization of starting materials, removing the traces of catalyst that affect the recovery of the product, and purification of the MIBT. The recovery of the important by-products that are obtained during the process is also found to be very important in any biochemical industrial process.

Therefore, there is a long-felt need for a production method capable of producing an alkyl-substituted aromatic hydrocarbon to obtain a highly selective yield of m-isobutyl toluene by achieving an excellent conversion rate of an aromatic hydrocarbon as a reaction substrate.

### OBJECTIVES OF THE INVENTION

The objective of the present invention is to provide a process for the synthesis of m-isobutyl toluene (MIBT) composition having a good yield.

Another objective of the present invention is to incorporate the reactants in an appropriate ratio that reduces the reaction time and thereby reducing overall reaction time for the process of obtaining an optimized yield of MIBT in side-chain alkylation of aromatic compounds.

### SUMMARY

This summary is not intended to identify essential features of the claimed subject matter, nor is it intended for use in determining or limiting the scope of the claimed subject matter. This summary is provided to introduce concepts related to the composition of catalysts for the alkylation of aromatic hydrocarbons, and the concepts are further described below in the detailed description.

The present disclosure relates to a process for the synthesis of m-isobutyl toluene (MIBT) comprising various steps. The process may comprise a step of adding a first amount of dry m-xylene and a K₂CO₃ carrier, to a first reactor forming a slurry mixture. The process may comprise a step of adding a predetermined molten sodium metal catalyst and a suspension agent to the slurry mixture within a definite time period. The process may comprise a step of transferring the slurry mixture comprising the sodium metal catalyst to a second reactor followed by adding a predetermined amount of propylene and an excess dry m-xylene to the second reactor heated to a predetermined temperature range. The process may comprise a step of reacting the slurry mixture, propylene, and m-xylene to obtain a crude-MIBT product. The process may comprise a step of removing the sodium catalyst from the crude-MIBT by enabling water washing in a neutralizer, and further recovering propane gas. The process may comprise a step of separating the aqueous layer and the organic layer in a separator unit, wherein the organic layer comprises crude-MIBT, and hydrocarbon by-products. The process may further comprise a step of recovering a purified form of MIBT by distillation mechanism having purity 99.9% and yield up to 40%.

### BRIEF DESCRIPTION OF DRAWINGS

The detailed description of the drawings is outlined with reference to the accompanying figures. In the figures, the left-most digit (s) of a reference number identifies the Figure in which the reference number first appears. The same numbers are used throughout the drawings to refer like features and components.

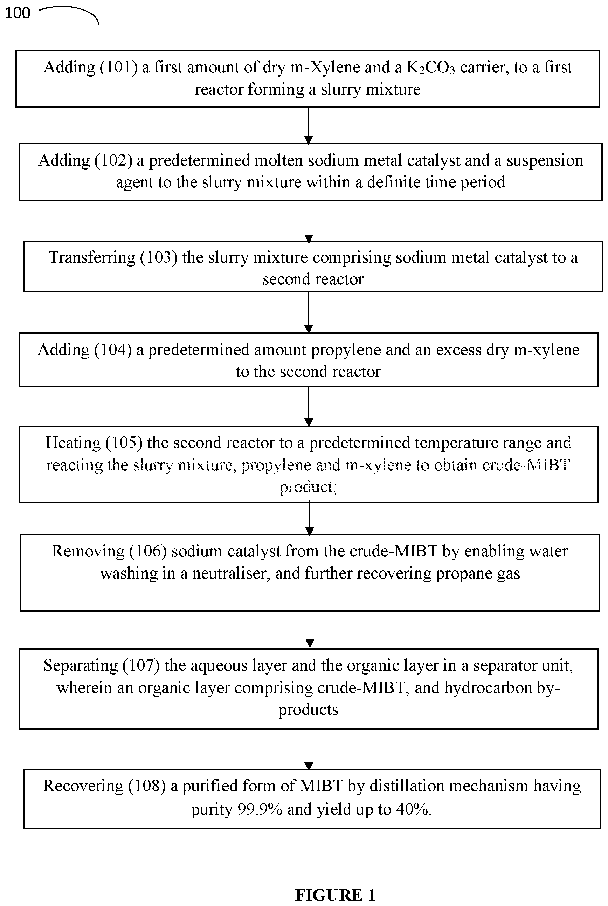

Figure 1 depicts a process flow chart (100) for the m-isobutyl toluene (MIBT), in accordance with an embodiment of the present subject matter.

### DETAILED DESCRIPTION

Reference throughout the specification to "various embodiments," "some embodiments," "one embodiment," or "an embodiment" means that a particular feature, structure, or characteristic described in connection with the embodiment is included in at least one embodiment. Thus, appearances of the phrases "in various embodiments," "in some embodiments," "in one embodiment," or "in an embodiment" in places throughout the specification are not necessarily all referring to the same embodiment. Furthermore, the particular features, structures, or characteristics may be combined in any suitable manner in one or more embodiments.

The words "comprising," "having," "containing," and "including," and other forms thereof are intended to be equivalent in meaning and be open-ended in that an item or items following any one of these words is not meant to be an exhaustive listing of such item or items or meant to be limited to only the listed item or items.

It must also be noted that the singular forms "a," "an," and "the" include plural references unless the context dictates otherwise. Although any methods similar or equivalent to those described herein can be used in the practice or testing of embodiments of the present disclosure, the exemplary methods are described. The disclosed embodiments are merely exemplary of the disclosure, which may be embodied in various forms. Various modifications to the embodiment may be readily apparent to those skilled in the art, and the generic principles herein may be applied to other embodiments. However, one of ordinary skill in the art may readily recognize that the present disclosure is not intended to be limited to the embodiments illustrated but is to be accorded the widest scope consistent with the principles and features described herein. The detailed description of the invention will be described hereinafter referring to accompanying drawings.

The present disclosure relates to an alkylation process to produce an alkyl-substituted aromatic hydrocarbon by alkylating the aromatic hydrocarbon at the meta-position by using a catalyst coated on a carrier material. The specific solid base, an alkaline earth metal compound, and an alkali metal hydride exhibit remarkably high alkylation activity, and efficiently use the desired alkyl-substituted aromatic hydrocarbon with a small amount of catalyst. The catalyst was found to be easy to be separated from the reaction product and furthermore destructed by dissolving in the water.

In one embodiment, referring to Figure 1, the present disclosure relates to a process (100) for the synthesis of m-isobutyl toluene (MIBT). As shown, the process comprises a step of adding (101) a first amount of dry m-xylene and a K₂CO₃ carrier, to a first reactor forming a slurry mixture.

The process (100) may further comprise a step of adding (102) a predetermined molten sodium metal catalyst and a suspension agent to the slurry mixture within a definite time period. The process (100) may further comprise a step of transferring (103) the slurry mixture comprising the sodium metal catalyst to a second reactor. The step of transferring (103) is followed by a step of adding (104) a predetermined amount of propylene and an excess dry m-xylene to the second reactor. Further, the process (100) may comprise a step of heating (105) the second reactor to a predetermined temperature range and reacting the slurry mixture, propylene and m-xylene to obtain a crude-MIBT product. The process may further comprise a step of removing (106) the sodium catalyst from the crude-MIBT by enabling water washing in a neutralizer, and further recovering propane gas. The process may comprise a step of separating (107) the aqueous layer and the organic layer in a separator unit, wherein the organic layer comprises crude-MIBT, and hydrocarbon by-products. The process may further comprise a step of recovering (108) a purified form of MIBT by distillation mechanism having purity of 99.9% and yield up to 40%.

Further, the present disclosure relates to a novel method for side-chain alkylation of an alkyl-substituted aromatic hydrocarbon with an aliphatic mono-olein. In particular, high conversion of side-chain alkylation of m-xylene with propylene is obtained. The present disclosure aims to provide a novel method for efficiently synthesizing m-isobutyl toluene.

In one embodiment, the process (100) may comprise as step of drying of m-xylene in a vacuum dryer wherein the initial moisture content of the m-xylene is in the range of 70 to 140 PPM, preferably in the range of 90 to 120 PPM, and more preferably in the range of 100 -125 PPM. In an embodiment, in the step of drying the moisture, content of m-xylene was removed by a vacuum dryer with a molecular sieve adsorber with a controlled flow rate to obtain dry m-xylene.

In one embodiment, the present disclosure relates to a process (100) for the synthesis of m-isobutyl toluene (MIBT) comprising a first step of adding (101) a first amount of the said dry m-xylene and a potassium carbonate (K₂CO₃) carrier, to a first reactor forming a slurry mixture.

In one embodiment of the present disclosure, a slurry mixture comprising a catalyst carrier, an alkali metal catalyst, and an organic solvent is proposed herein, wherein the alkali metal catalyst in molten form is coated over the catalyst carrier and suspended in the organic solvent. The catalyst composition may be used for alkylating aromatic hydrocarbons and the said catalyst composition may be composed of a carrier material and alkali metal catalyst.

In one embodiment of the present disclosure, the said organic solvent may comprise at least one aromatic hydrocarbon selected from but not limited to m-xylene, p-xylene, n-propylene, and liquified isobutene. The said organic solvent is employed in the catalyst slurry for the formation of a uniform mixture of the catalyst, and the catalyst carrier. This solvent is usually inert and does not contribute to the catalysis reaction. However, the solvent may be a starting material in the reaction of alkylation of aromatic compound which is also used as suspending solvent on a catalyst composition. In one embodiment, the said organic solvent is selected as but not limited to m-xylene.

In another embodiment, the solid base used in the process (100) is an alkaline earth metal compound selected from one or more of magnesium oxide, magnesium hydroxide, magnesium carbonate, calcium oxide, calcium hydroxide, and calcium carbonate, potassium compound containing at least one of potassium hydroxide and potassium carbonate as well as the composition containing metallic sodium. Specifically, the solid base is a metal sodium in an inert gas atmosphere.

In one embodiment of the present disclosure, the alkali metal catalyst comprises at least one of sodium, lithium, potassium, rubidium, cesium, NaK, and Na₂O. The said alkali metals used as catalysts are transition metals that are highly reactive and electropositive. These metals tend to lend or take electrons from other molecules easily, thus increasing the rate of reaction. Alkali metals are employed in the reduction of organic compounds and the preparation of many commercial compounds. More preferably, the alkali metal catalyst may be selected as sodium.

In a related embodiment of the present disclosure, the catalyst carrier comprises at least one of potassium carbonate, potassium bicarbonate, sodium carbonate, and sodium bicarbonate. The catalyst carrier is typically a solid material kept under certain conditions to carry, support, or load the catalyst which is usually inert but can contribute to catalytic activity. Preferably, the catalyst carries potassium carbonate.

In an embodiment of the process (100), potassium carbonate is in the range of 0.6 to 1.1 mm, preferably 07 to 1.0 mm. and more preferably in the range of 0.8 to 0.9 mm.

In one embodiment, the said catalyst carrier may be present in any form not limited to powder, pellets, or granules. More preferably, the said catalyst carrier is in a form of a crushed fine powder providing a larger surface area. The larger surface area offers more surface area to form active sites thus leading to greater activity.

In an embodiment, the process for the synthesis of MIBT comprises the ratio of the amount of sodium, potassium carbonate (K₂CO₃), and the suspension agent may be in the range of 5:40:1 to 10:50:1, and preferably in the range of 7:50:1 to 9:60:1, and more preferably in the ratio of 6.2:53: 1.

In a preferred embodiment, the process for the synthesis of MIBT wherein the suspension agent may be selected from fatty acids such as oleic acid, tall oil, and stearic acid.

In an embodiment, the process (100) may comprise addition of a predetermined range of 7.5 to 13.6 moles of molten sodium metal catalyst; preferably 9 to 13 moles of molten sodium metal catalyst and a suspension agent to the slurry mixture within a period range of 5 minutes to 1 hour 30 minutes, preferably 10 minutes to 1 hour 10 minutes, and more preferably 20 minutes to 1 hour.

In an embodiment, the process (100) may comprise a step transferring (103) the slurry mixture comprising sodium metal catalyst to a second reactor.

The aromatic hydrocarbon in the present embodiment is an aromatic hydrocarbon having an alkyl group having a hydrogen atom at the alpha-position. The alpha position in the present embodiment is a carbon position on the alkyl group and is a position adjacent to the carbon on the aromatic hydrocarbon ring to which the alkyl group is bonded.

Examples of the aromatic hydrocarbon include toluene, ethylbenzene, n-propylbenzene, isopropylbenzene, o-xylene, m-xylene, p-xylene, o-diethylbenzene, m-diethylbenzene, p-diethylbenzene, mesitylene, pseudocumene, 1,2,3,5-tetramethylbenzene, 1,2,4,5-tetramethyl benzene, pentamethyl benzene, hexamethyl benzene, 1-methyl naphthalene, 2 -Methylnaphthalene, dimethyl naphthalene, tetrahydro naphthalene, indane, 2-methylpyridine, 4-methylpyridine and the like. These aromatic hydrocarbons may be used individually by 1 type, and may be used in combination of 2 or more type. In one embodiment, preferably m-xylene may be used as the starting material and the solvent.

The alkylation in this embodiment indicates that the meta-position hydrogen atom of the alkyl group in the aromatic hydrocarbon is substituted with an alkyl group by reaction with an alkene. Examples of the alkene in the present embodiment include linear or branched alkenes having 2 to 20 carbon atoms. Specifically, ethylene, propylene, 1-butene, 2-butene, isobutylene, 1-pentene, 2- Pentene, 1-hexene, 2-hexene, 3-hexene, 1-heptene, 2-heptene, 3-heptene, octene, nonene, 3-methyl-1-butene, 2-methyl-2-butene, 3-methyl- Examples thereof include 1-pentene and 3-methyl-2-pentene. These alkenes may be used individually by 1 type and may be used in combination with 2 or more types. Among these alkenes, propylene is preferable.

In an embodiment, the process may comprise a step of adding (104) a predetermined amount of propylene and an excess dry m-xylene to the second reactor.

The mole ratio of alkene to aromatic hydrocarbon in these coupling reactions varies with the particular reactants employed and the products desired, particularly since the aromatic hydrocarbon may have one or more active hydrogens, and it may be desired to react the alkene with only one or with more than one active hydrogen in the aromatic hydrocarbon. It is frequently preferred to employ the reactants in the stoichiometric amounts appropriate for the preparation of the desired product. However, either reactant can be used in excess.

In an embodiment, the ratio of the amount of m-xylene and propylene is 1:1 to 15:1. In a preferred embodiment, the ratio of m-xylene and propylene is 2:1 to 10:1. In a more preferred embodiment, the ratio of the amount of m-xylene and propylene is 0.2 to 1.2 and preferably 0.4 to 0.9.

In an embodiment, the process (100) further comprises heating (105) the second reactor to a temperature range of 180-220°C and reacting the slurry mixture, propylene, and m-xylene to obtain a crude-MIBT product in the crude MIBT stream.

In an embodiment, the said reactor crude MIBT stream comprises an admixture of unreacted m-xylene, propane, crude MIBT, low hydrocarbons (LHC), and high hydrocarbons (HHC) formed in the reaction system.

In another embodiment, the process (100) further comprises removing (106) sodium catalyst from the crude-MIBT by enabling water washing in a neutraliser, and further recovering propane gas through a gas holder directed to utility.

In an embodiment, the process (100) for the synthesis of MIBT further comprises separating (107) the aqueous layer and the organic layer in a separator assembly, wherein an organic layer comprising crude-MIBT, and hydrocarbon by-products by overflow mechanism.

In one aspect of the embodiment, at least one MIBT enriched organic stream which is free from the catalyst or has low catalyst impurities. may be separated in the separation assembly.

In an embodiment, the said separated aqueous layer is directed to a wastewater tank from the separator assembly and the separated organic layer is transferred to the LHC column.

In an embodiment, the process (100) comprises recovering (108) a purified form of MIBT by a distillation mechanism having a purity of 99.9% and yield up to 40%.

In another embodiment, the MIBT is checked in an internal inspection tank before storing in the MIBT storage tank.

In the present disclosure of the process (100) for the synthesis of MIBT, the aromatic hydrocarbon is preferably m-xylene, and the alkene is preferably propylene.

Furthermore, in the production method of the present embodiment, the alkyl-substituted aromatic hydrocarbon is preferably m-isobutyl toluene, and the alkene is preferably propylene.

In one embodiment, the present disclosure improves the conversion rate of the aromatic hydrocarbon that is the reaction substrate and can obtain the target alkyl-substituted aromatic hydrocarbon with high selectivity and high yield, which is industrially advantageous and can be manufactured. In addition, alkyl-substituted aromatic hydrocarbons useful as intermediate raw materials such as pharmaceuticals and fragrances can be obtained by the production method of the present disclosure.

### Experimental Details:

Hereinafter, the present disclosure will be described in detail by way of examples, but the present disclosure is not limited to these examples.

### Example 1: Yield of m-isobutyl toluene (MIBT) using different ratios of propylene and m-xylene with catalyst slurry

| Exp. No. | Condition | **Moles of suspending solvent (m-xylene)** | **Moles of propylene** | **Mole ratio** | Catalyst carrier (K₂CO₃) | Catalyst (Sodium) | MIBT % |
|---|---|---|---|---|---|---|---|
| 1 | No Change | 7.60 | 13.10 | 0.58 | 106 | 12.5 | 37.19 |
| 2 | No Change | 7.60 | 13.10 | 0.58 | 106 | 10.9 | 36.97 |
| 3 | No Change | 7.60 | 13.10 | 0.58 | 106 | 10.7 | 35.74 |
| 4 | Higher moles of propylene charged | 7.60 | 15.48 | 0.49 | 106 | 10.5 | 34.1 |
| | Higher moles of propylene charged | 7.60 | 15.48 | 0.49 | 106 | 11.6 | 37.19 |
| 5 | Less qty. of catalyst | 7.60 | 5.95 | 1.28 | 75 | 7.5 | 9.42 |
| 6 | No sodium added | 7.60 | 2.26 | 3.36 | 106 | 0 | 0.68 |
| 7 | Uncrushed K₂CO₃ | 7.60 | 4.52 | 1.68 | 106 | 10.5 | 14.63 |
| 8 | Higher Moles of xylene charged | 9.46 | 11.31 | 0.84 | 132 | 13.6 | 26.79 |

By referring to Table 1, it is evident that organic solvent (m-xylene), catalyst carrier (K₂CO₃), and alkali metal catalyst are taken in a defined form as discussed in various embodiments of the present disclosure. MIBT product recovery is improved when the ratio of m-xylene and propylene was varied with catalyst slurry. It was particularly observed that the ratio of moles of m-xylene: moles of propylene in a range of 0.4-0.9. A distinct difference in the yield of MIBT was noticed. Referring to the Example 1 within the ratio of moles of m-xylene: moles of propylene in a range of 0.4-0.9 the overall yield of MIBT with respect to m-xylene was achieved up to 40%.

### Example 2: Yield of m-isobutyl toluene (MIBT) using different catalyst suspension and additives

| Exp. No. | Condition | Moles of suspending solvent (m-xylene) | Catalyst carrier (K₂CO₃) | Catalyst (Sodium) | MIBT % |
|---|---|---|---|---|---|
| 1 | 2 g (oleic acid) O.A. | 7.60 | 106 | 12.4 | 37.19 |
| 2 | 2 g Tall Oil | 7.60 | 106 | 10.23 | 4.68 |
| 3 | 2 g stearic acid | 7.60 | 106 | 12.20 | 29.20 |

In one embodiment of the present disclosure, the said catalyst slurry is added with a defined ratio of m-xylene and propylene which improved the yield of the product of alkylation of aromatic hydrocarbons.

In another embodiment of the disclosure, the process (100) in accordance with the present disclosure may have the following advantages, including but not limited to:
- Improved synthesis of MIBT
- Improves quality and quantity of MIBT obtained
- Improvement in the product yield such the up to 40% MIBT is recovered.

Although implementations used for the synthesis of alkylated aromatic hydrocarbons have been described in language specific to structural features and/or processes, it is to be understood that the appended claims are not necessarily limited to the specific features or processes described. Rather, the specific features and processes are disclosed as examples of implementations for a composition of catalyst implemented thereon for the synthesis of alkylated aromatic hydrocarbons.

## Claims

1. A process (100) for the synthesis of m-isobutyl toluene (MIBT) comprising:
adding (101) a predetermined amount of dry m-Xylene and a potassium carbonate carrier, to a first reactor forming a slurry mixture;
adding (102) a predetermined molten sodium metal catalyst and a suspension agent to the slurry mixture within a definite time period;
transferring (103) the slurry mixture comprising sodium metal catalyst to a second reactor;
adding (104) a predetermined amount of propylene and an excess dry m-xylene to the second reactor;
heating (105) the second reactor to a predetermined temperature range and reacting the slurry mixture, propylene, and m-xylene to obtain crude-MIBT product;
removing (106) sodium catalyst from the crude-MIBT by enabling water washing in a neutralizer, and further recovering propane gas;
separating (107) the aqueous layer and the organic layer in a separator unit, wherein an organic layer comprising crude-MIBT, hydrocarbon by-products; and
recovering (108) a purified form of MIBT by distillation mechanism having a purity of 99.9% and yield up to 40%.

2. The process as claimed in claim 1, wherein the ratio of amount m-xylene and propylene is 0.2-1.2.

3. The process as claimed in claim 1, wherein the ratio of the amount of sodium, potassium carbonate, and the suspension agent is 7:50:1 to 9:60: 1.

4. The process as claimed in claim 1, wherein the sodium metal catalyst is added within a time period range of 20 minutes to 1 hour.

5. The process as claimed in claim 1, wherein the second reactor is heated to a temperature range of 180 to 220 ° C.

6. The process as claimed in claim 1, wherein the propane gas is removed from neutralizer through gas holder to utility.

7. The process as claimed in claim 1, wherein the hydrocarbon by-products are present in the organic layer.

8. The process as claimed in claim 1, wherein the suspension agent is selected from fatty acids such as oleic acid, tall oil, and stearic acid.

9. The process as claimed in claim 1, wherein the initial moisture content of the m-xylene is in the range of 100 -125 PPM.

10. The process as claimed in claim 1, wherein the moisture content of m-xylene is removed by vacuum dryer with molecular sieve adsorber with a controlled flow rate to obtain dry m-xylene.

11. The process as claimed in claim 1, wherein potassium carbonate is in the range of 0.8 to 0.9 mm.

12. The process as claimed in claim 1, wherein the potassium carbonate is in a form of crushed powder.

13. The process as claimed in claim 1, wherein the catalyst preparation time cycle comprises m-xylene feeding for 5 minutes, potassium carbonate and Na charging for 25 minutes, heating for 30 minutes, digestion for 15 minutes, and charging the reactor for 15 minutes.
